# EUROPÄISCHE PATENTSCHRIFT

(11) **EP 2 801 334 B1**
(45) Veröffentlichungstag und Bekanntmachung des Hinweises auf die Patenterteilung: **29.05.2024**
(21) Anmeldenummer: 14167407.7
(22) Anmeldetag: 07.05.2014
(51) Int. Cl.: A61B 46/00

(54) **Dilatationsvorrichtung und ausdehnbarer Überzug für ein Dilatationsinstrument**
Dilation device and expandable sleeve for a dilation instrument
Dispositif de dilatation et revêtement extensible pour un instrument de dilatation

(30) Priorität: 08.05.2013 DE 102013104789
(43) Veröffentlichungstag der Anmeldung: 12.11.2014
(73) Patentinhaber: Karl Storz SE & Co. KG, 78532 Tuttlingen (DE)
(72) Erfinder: Bacher, Uwe, 78532 Tuttlingen (DE); Blocher, Martin, 78532 Tuttlingen (DE); Schmidberger, Jochen, 78532 Tuttlingen (DE); Unger, Tobias, 78532 Tuttlingen (DE)

(56) Entgegenhaltungen:
- EP-A1- 2 245 990
- WO-A1-2005/058147
- DE-U1-202008 005 879
- DE-U1-202012 104 533
- US-A- 1 271 456
- US-A- 5 178 133
- US-A1- 2006 135 981

## Beschreibung

Die nachfolgende Erfindung bezieht sich auf einen Überzug für ein Dilatationswerkzeug und auf ein Dilatationsinstrument.

Dilatationsinstrumente werden u. a. in der endoskopischen HNO-Chirurgie, aber auch in anderen Fachgebieten der Medizin eingesetzt. Mit ihnen werden krankhafte Verengungen beseitigt, indem ein ausdehnbarer Abschnitt in den Bereich der Verengung eingeführt und der Abschnitt aufgeweitet wird. Eine einfache Lösung zur Aufweitung ist es, einen Ballonkatheter zu verwenden, was nachteilig ist, wenn der Ballon platzt und "Pumparbeit" vom Operateur verlangt, wenn ein Gas eingesetzt wird. Kommt eine inkompressible Flüssigkeit zum Einsatz, so ist wiederum auf deren Bioverträglichkeit zu achten. Es sind auch rein mechanische Dilatationsinstrumente bekannt, die jedoch meist nicht ideal aufweitbar sind, dafür aber mit geringeren Handbewegungen eine feinfühlige Kraftrückmeldung zulassen.

Mechanische Dilatationsinstrumente weisen häufig den Nachteil auf, dass sich zwischen den Spreizbacken im gespreizten Zustand vergleichsweise weite Spalte bilden, was aufgrund zu starker Druckbelastung und durch Einklemmen mit zu einer erhöhten Traumagefahr beiträgt. Aufgrund dessen wurden für mechanische Dilatationsinstrumente Überzieher entwickelt, die diese Spalte ausgleichen, die Krafteinleitung in das Gewebe homogenisieren und das Eindringen von Gewebe in die Dilatationsmechanik verhindern sollen.

Ein Dilatationsinstrument mit einem solchen Überzieher sowie ein Verfahren und eine Vorrichtung zum Aufbringen des Überziehers ist aus der EP 2 412 315 A2 bekannt. Dort wird der Überzieher in der Vorrichtung gehalten und an seinem proximalen Ende aufgeweitet. Die Vorrichtung weist eine kegelförmige Einführhilfe für das ausdehnbare Ende des Dilatationsinstrument auf, so dass es leichter in den Überzieher eingeführt werden kann. Der Überzieher dort erstreckt sich lediglich über den ausdehnbaren Abschnitt, in dem auch die Dilatationsmechanik angeordnet ist. Es kann passieren, dass der Überzieher bei Betätigung der Dilatationsmechanik ungewollt von dem ausdehnbaren Ende herunter rutscht.

Die Gebrauchsmusterschrift DE 20 2012 104 533 U1 offenbart einen Bezug für Wundhaken und ähnliche Instrumente. Aufgrund seiner Elastizität ist er in der Lage, sich an unterschiedlich große Instrumente anzupassen. Der Bezug soll das unerwünschte Abrutschen des Wundhakens vom gehaltenen Gewebe verhindern. Er wird mittels eines Befestigungsbands am Instrument gehalten.

Das Gebrauchsmuster DE 20 2008 005 879 U1 lehrt einen Wundhakenbezug aus langgestrecktem Rundstrickstoff, welcher entlang seiner Längsachse verdreht und auf sich selber zurückgestülpt wird. Dadurch umschließt er den Wundhaken doppellagig und weist eine erhöhte Stabilität und Saugfähigkeit auf. Das Herunterrutschen vom Wundhaken wird durch ein angenähtes Befestigungsband verhindert.

Das US-Patent 1,271,456 offenbart ein Instrument zum Aufdehnen des Rektums mit einer Vielzahl von radial auswölbbaren Lamellen. Diese Lamellen befinden sich unter einem Überzug aus Gummi.

Die Patentschrift US 5,178,133 A lehrt einen Retraktor für die Laparoskopie mit auslenkbaren Endabschnitten. Diese Endabschnitte werden von einem aufweitbaren Überzug aus einem elastischen Membranmaterial wie Latex, Gummi umgeben.

Diese Aufgabe wird durch eine Dilatationsvorrichtung mit den Merkmalen des unabhängigen Anspruchs 1 gelöst.

Weiterbildungen sind jeweils in den Unteransprüchen ausgeführt.

Die erfindungsgemäße Dilatationsvorrichtung weist gemäß einer ersten Ausführungsform ein Dilatationsinstrument mit einem distalen radial ausdehnbaren Abschnitt, der über einen länglichen Schaft mit einem Handhabeabschnitt gekoppelt ist, und einen aufweitbaren Überzug für den Ausdehnungsabschnitt des Dilatationsinstruments, der ein geschlossenes distales Ende und ein offenes proximales Ende hat, auf. Der Überzug hat einen Schaftabschnitt, der sich entlang des Schafts des Dilatationsinstruments erstreckt. Das offene proximale Ende des Überzugs ist lösbar mit einem distalen Ende des Handhabeabschnitts des Dilatationsinstruments gekoppelt.

Proximal und distal ist hierin in Bezug zu einer Person, die die Dilatationsvorrichtung benutzt, zu verstehen. Proximal ist demnach eine dieser Person nahe gelegene Position und distal eine der Person fern gelegene Position.

Der Überzug, der sich über einen Großteil der Länge des Schafts des Dilatationsinstruments erstreckt oder gar über dessen komplette Länge, bietet im Vergleich zu bekannten Überzügen, die nur über den Ausdehnungsabschnitt gestreift sind, Vorteile: So kann der Überzug auch bei großer Ausdehnung des Ausdehnungsabschnitts nicht unbeabsichtigt herunterrutschen; eventuell wird auch eine Längsdehnung stattfinden, jedoch ist der Überzug sicher am Handhabeabschnitt befestigt. Die radiale Ausdehnbarkeit bzw. Elastizität des Überzugs im Ausdehnungsabschnitt soll mindestens so dimensioniert sein, dass er der Ausdehnung des Dilatationsinstruments ohne Beschädigung folgen kann, es kann dafür auch eine Sicherheitsreserve vorgesehen werden.

Der Überzug kann daher vorteilhaft ein Einwegüberzug sein, jedoch ist nicht ausgeschlossen, dass der Überzug auch mehrmals verwendet werden kann, was insbesondere sinnvoll sein kann, wenn wertvolle Werkstoffe zum Einsatz kommen. Die Kopplung des Überzugs mit dem Handhabeabschnitt des Dilatationsinstruments kann indirekt oder direkt erfolgen; eine direkte Verbindung kann etwa erreicht werden, indem der Überzug in eine Kopplungsvorrichtung am Handhabeabschnitt eingelegt wird, etwa eine Art Spannring oder Spannhaken. Eine indirekte Kopplung kann dadurch erreicht werden, dass der Überzug mit einem Zwischenelement zur Kopplung, etwa einer Mutter oder einem Schnellverschlusselement verbunden wird, und dieser dann mit dem Handhabeabschnitt gekoppelt wird. Die erfindungsgemäße Dilatationsvorrichtung kann grundsätzlich mit mechanischen, hydraulischen, pneumatischen oder elektrischen Dilatationsinstrumenten betrieben werden. Ferner ist der Überzug nicht auf bestimmte Schaftdurchmesser, Schaftlängen oder Durchmesser des Ausdehnungsabschnittes des Dilatationsinstruments beschränkt.

Der erfindungsgemäße Überzug besteht aus aus einem Netzmaterial , etwa einem Drahtnetzmaterial, das vorteilhaft aus einem biokompatiblen Edelstahl gefertigt sein kann.

Bei einem Netzmaterial kann die Ausdehnung des Überzugs nicht nur allein durch elastische Dehnung der Drähte erreicht werden, sondern auch - und unter deutlich geringerem Krafteinsatz - durch gegenseitiges Abgleiten bzw. Verschieben der Drähte. Die genannten Werkstoffe sind nicht beschränkend zu verstehen. Weitere Werkstoffe für das Netzmaterial sind denkbar, so etwa Polymere.

In einer noch weiteren Ausführungsform kann der Schaftabschnitt des Überzugs am offenen proximalen Ende mit einer Kopplungshülse verbunden sein, insbesondere mit der Kopplungshülse verklebt sein. Zusätzlich kann mit dem fernen Ende des Handhabeabschnitts des Dilatationsinstruments eine Kopplungsvorrichtung verbunden sein, insbesondere lösbar verbunden und besonders vorteilhaft über ein Gewinde verbunden. Die Kopplungshülse des Überzugs kann dabei lösbar mit der Kopplungsvorrichtung des Dilatationsinstruments gekoppelt sein.

Es handelt sich in dieser Ausführungsform um eine indirekte Kopplung, da seitens des Überzugs die Kopplungshülse und auf Seiten des Dilatationsinstruments die Kopplungsvorrichtung "zwischengeschaltet" ist. Mit dieser Art der Kopplung kann der Überzug deutlich schneller als bei direkter Kopplung mit dem Dilatationsinstrument gekoppelt und von diesem entkoppelt werden. Bei indirekter Kopplung ist vorteilhaft, dass der Kopplungs- und der Entkopplungszustand explizit vorgegeben sind; die Kopplungshülse und Kopplungsvorrichtung können nur auf vorgegebene Weise in Eingriff gebracht werden. Bei direkter Kopplung wird der Überzug in die Spannvorrichtung, etwa eine Spannzange, ein Spannring oder ein Spannhaken eingehängt, wobei die Gefahr besteht, dass beim Koppeln Benutzerfehler unterlaufen, die letztlich zum unbeabsichtigten Lösen des Überzugs führen könnten. Der Überzug kann über die Kopplungshülse gezogen sein, mit der äußeren Mantelfläche der Kopplungshülse verklebt sein aber auch zusätzlich mit formschlüssigen Haltemitteln, etwa Haken, die mit den Strukturabmessungen des Überzugs korrespondieren, gehalten werden. Die Kopplungsvorrichtung kann besonders vorteilhaft die gleiche Außenquerschnittsform und die gleichen Abmessungen haben, wie auch der Handhabeabschnitt des Dilatationsinstruments, so dass sich die Kopplungsvorrichtung harmonisch in das Gesamtbild des Instruments einfügt und auch ergonomisch keine störenden Übergänge verursacht.

Des Weiteren kann die Kopplungshülse ein Bajonettverschlusselement aufweisen, das insbesondere eine im Wesentlichen L-förmige Einführausnehmung haben kann, die sich von einer proximalen Stirnfläche der Kopplungshülse längsaxial in der Wand der Kopplungshülse erstreckt. Am geschlossenen Ende der L-förmigen Einführausnehmung kann eine Sperrausnehmung vorliegen. Zusätzlich kann die Kopplungsvorrichtung zumindest eine sich radial nach innen erstreckende Sperrnase haben, deren Abmessungen mit der Einführausnehmung der Kopplungshülse korrespondieren. Die Sperrnase der Kopplungsvorrichtung wird im Sperrzustand von der Sperrausnehmung der Kopplungshülse aufgenommen.

Die Kopplungshülse und die Kopplungsvorrichtung bilden in dieser Ausführungsform einen Bajonettverschluss, der selbstverständlich auch in einen Öffnungszustand überführt werden kann. Wie auch bekannte Bajonettverschlüsse mit L-förmiger Einführausnehmung bzw. Führungsnut, wird die Kopplungshülse dazu längsaxial bewegt, um den Eingriff in der Sperrausnehmung zu überwinden und dann durch Drehen, gefolgt von längsaxialem Verschieben, aus der Einführausnehmung ausgefädelt werden. Die Kopplungshülse kann vorteilhaft aus einem spritzbaren Kunststoff bestehen, da so auch komplexe Geometrien zu überschaubaren Kosten herstellbar sind. Es ist aber hierdurch nicht ausgeschlossen, dass auch andere Werkstoffe wie Metalle oder Keramiken zum Einsatz kommen können, wenn etwa erhöhte Hygieneanforderungen oder eine höhere Verschleißbeständigkeit gewünscht sind. Die nötige längsaxiale Vorspannkraft der Kopplungshülse, die die Sperrnase in die Sperrausnehmung drückt, kann vorteilhaft durch den Überzug selbst erzeugt werden, nämlich, indem der Überzug in ungedehntem Zustand etwas zu kurz ist, um über den Schaft gezogen zu werden und erst mit geringer längsaxialer Dehnung in Eingriff mit der Einführausnehmung gebracht werden kann. Hierbei kann es vorteilhaft möglich sein, dass die Kopplungshülse zweiteilig ausgeführt ist: Sie kann etwa aus einer drehbaren Innenhülse, die in Eingriff mit der Kopplungsvorrichtung gebracht werden kann, und einer drehfest mit dem schlauchartigen Schaft verbundene Außenhülse bestehen, da so vermieden werden kann, dass beim Betätigen des Bajonettverschlusses der schlauchartige Schaft mit verdreht wird.

Ferner kann zwischen einer proximalen Stirnfläche der Kopplungshülse und einer distalen Stirnfläche der Kopplungsvorrichtung ein Federelement angeordnet sein, wobei eine Druckfeder vorteilhaft ist, und eine Polymerscheibe, wie ein O-Ring, besonders geeignet ist.

Durch Anbringung des Federelements kann der Bajonettverschluss gesichert werden, ohne Zug auf den gesamten Überzug aufbringen zu müssen, was die Belastung des Überzugs reduziert. Wird zum Schließen des Bajonettverschlusses die Sperrnase durch die Einführausnehmung bis zur Sperrausnehmung bewegt, das Federelement reversibel komprimiert und beim Einrasten der Sperrnase in die Sperrausnehmung wieder teilweise entlastet, wobei noch immer so viel Vorspannung vorhanden sein muss, dass die Sperrnase sicher gehalten werden kann.

Darüber hinaus kann die Kopplungsvorrichtung einen Aufnahmeraum für die Kopplungshülse des Überzugs haben, der sich längsaxial von einer distalen Stirnfläche der Kopplungsvorrichtung in Richtung ihres proximalen Endes erstreckt. Die Kopplungshülse ist zumindest entlang ihres proximalen Endabschnitts darin aufgenommen. Ferner kann an den Aufnahmeraum proximal eine Auflagestirnfläche angrenzen an der sich die Kopplungshülse abstützt.

Dadurch, dass die Kopplungshülse wenigstens teilweise von der Kopplungsvorrichtung aufgenommen ist, wird vorgebeugt, dass Blut, Gewebe oder anderer Schmutz nicht in die Kopplungshülse eindringt und deren Funktion blockiert, während der über die Hülse gestreifte Überzug das Eindringen solcher Stoffe vom distalen Ende her verhindert.

Gemäß einer weiteren Ausführungsform kann an der Kopplungsvorrichtung umfänglich benachbart zu der Sperrnase wenigstens eine sich radial nach außen erstreckende Montageausnehmung vorliegen, wobei insbesondere zwei Montageausnehmungen beidseitig der Sperrnase vorliegen können. Alternativ oder zusätzlich kann die Kopplungsvorrichtung am distalen Ende des Aufnahmeraums eine Einführanfasung aufweisen.

Die Einführanfasung soll das Einführen der Kopplungshülse in den Aufnahmeraum der Kopplungsvorrichtung erleichtern, indem der Öffnungsraum sich in distaler Richtung "aufweitet". Der Benutzer muss also beim Koppeln weniger aufmerksam sein; ihm bleibt dadurch mehr Aufmerksamkeit für andere Tätigkeiten. Zudem wird das Koppeln beschleunigt, da ein Verkanten der Kopplungshülse an Bunden oder Vorsprüngen verhindert werden kann. Die Montageausnehmungen dienen einer vereinfachten Montage und Demontage der Kopplungsvorrichtung am Dilatationswerkzeug, etwa wenn diese gereinigt werden muss. Unter Verwendung eines Spezialwerkzeugs, das mit den Montageausnehmungen korrespondiert, kann die Kopplungsvorrichtung mit hinreichend hohem Drehmoment angezogen werden und ohne große Anstrengung wieder gelöst werden.

Diese und weitere Vorteile werden durch die nachfolgende Beschreibung unter Bezug auf die begleitenden Figuren dargelegt.

Der Bezug auf die Figuren in der Beschreibung dient der Unterstützung der Beschreibung und dem erleichterten Verständnis des Gegenstands. Gegenstände oder Teile von Gegenständen, die im Wesentlichen gleich oder ähnlich sind, können mit denselben Bezugszeichen versehen sein. Die Figuren sind lediglich schematische Darstellungen von Ausführungsbeispielen der Erfindung.

Es zeigen:
Fig. 1 eine perspektivische Ansicht des Überzugs,
Fig. 2eine perspektivische Ansicht des Überzugs mit teilausgedehntem Ausdehnungsabschnitt,
Fig. 3eine perspektivische Ansicht des Überzugs mit voll ausgedehntem Ausdehnungsabschnitt,
Fig. 4eine perspektivische Teilansicht des Überzugs,
Fig. 5 eine Draufsicht auf ein Teil des Überzugs,
Fig. 6eine perspektivische Ansicht der Kopplungshülse,
Fig. 7eine perspektivische Ansicht der Kopplungsvorrichtung,
Fig. 8eine Draufsicht der Kopplungsvorrichtung,
Fig. 9ein Längsschnitt der Kopplungsvorrichtung
Fig. 10 eine perspektivische Ansicht des Dilatationswerkzeugs ohne Überzug,
Fig. 11 ein Längsschnitt des Dilatationswerkzeugs ohne Überzug,
Fig. 12 ein Längsschnitt des Dilatationswerkzeugs mit Überzug,
Fig. 13 ein Längsschnitt eines anderen Dilatationswerkzeugs mit Überzug,
Fig. 14 eine weitere Draufsicht eines Überzugs,
Fig. 15 eine Draufsicht des Details B der Fig. 14.

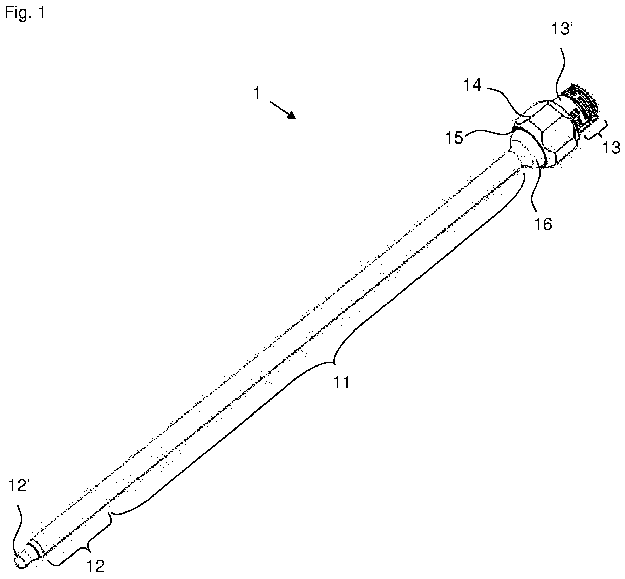

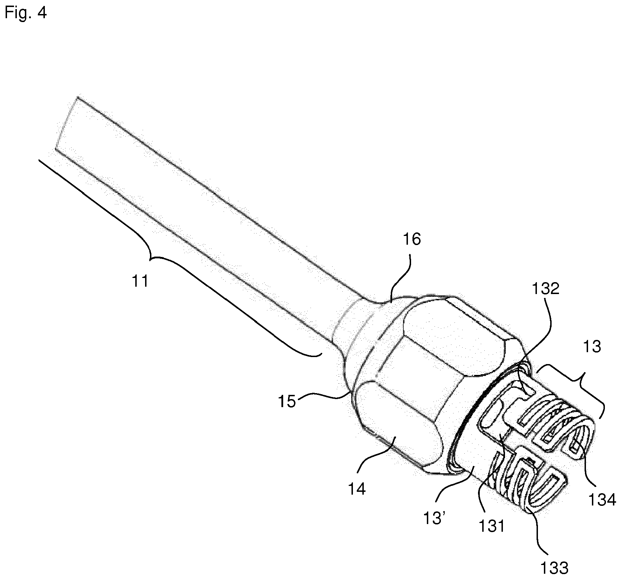

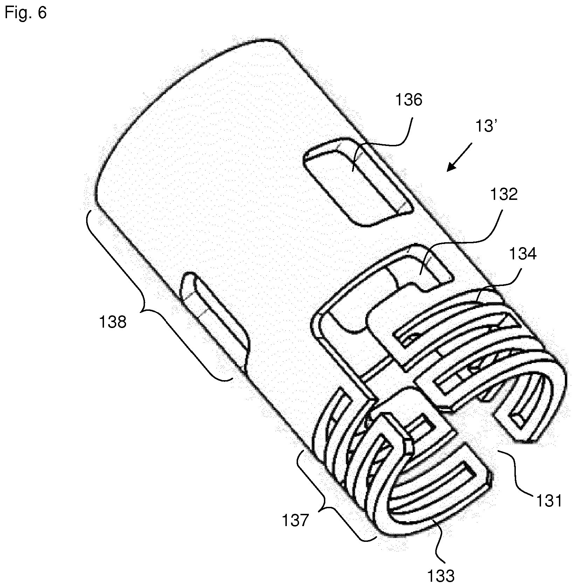

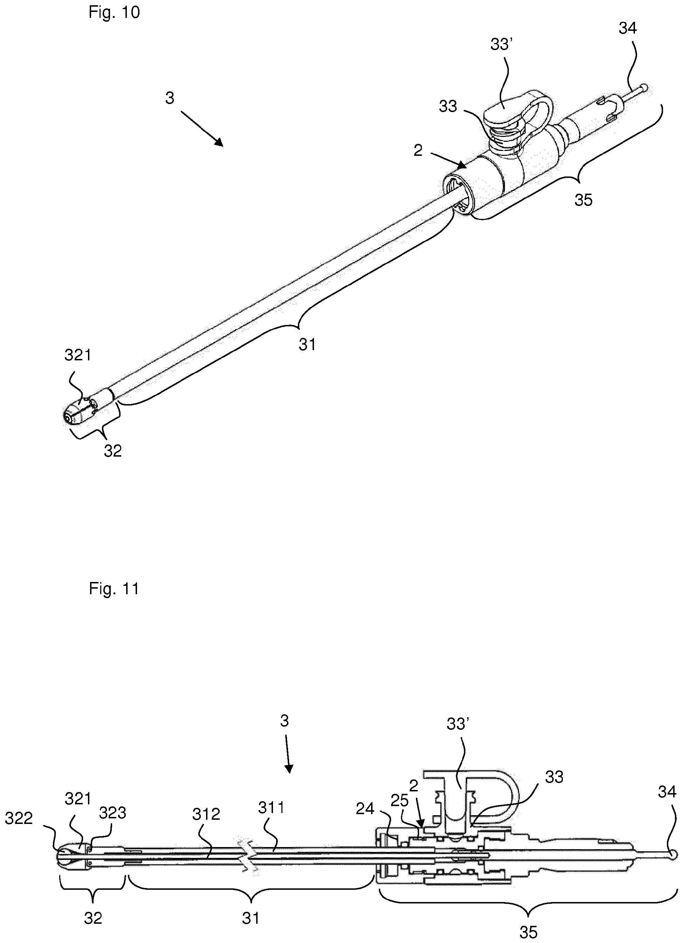

Der in Fig. 1 dargestellte Überzug 1 weist drei Abschnitte11, 12, 13 auf, einen proximalen Kopplungsabschnitt 13, einen mittleren Schaftabschnitt 11 und einen distalen Ausdehnungsabschnitt 12. Der Überzug 1 kann wie eine Haube über ein längliches Dilatationswerkzeug 3 (siehe Fig. 10) gezogen werden, dazu ist der Überzug 1 vom Kopplungsabschnitt 13 bis zur Spitze, dem Ausdehnungsabschnitt 12 offen. Im Kopplungsabschnitt 13 ist der Schlauch des Schaftabschnitts 11 über eine Kopplungshülse 13' gestreift und mit dieser verklebt. Die Klebestelle wird wiederum von einer Griffhilfshülse 14 abgedeckt, die drehfest mit der Kopplungshülse 13' verklebt ist, und zusätzlich auch noch formschlüssig gehalten wird durch ein Zusammenwirken einer Rastausnehmung und einer Rastnase (siehe Fig. 6). Der Schlauch besteht aus einem elastischen Netzmaterial, das sich radial um das mehrfache seines Ruhedurchmessers gedehnt werden kann ohne plastisch verformt oder gar zerstört zu werden, was an der Übergangsstelle 16 deutlich wird: Dort wird der Schlauch auf vergleichsweise kurzer längsaxialer Strecke stark aufgeweitet, so dass er in den Ringspalt zwischen Kopplungshülse 13' und Griffhilfshülse 14 geführt werden kann. Am distalen Ende hat der Überzug 1 eine Endhülse 12', die über die Spitze des Schlauchs geschoben ist und mit dem Schlauch 11' verklebt ist oder ähnlich wie eine Adernendhülse auch verquetscht sein kann. Der Kopplungsabschnitt 13 dient dazu, den Überzug 1 sicher aber dennoch schnell mit einem Dilatationsinstrument koppeln zu können. Dafür liegt im Kopplungsabschnitt 13 ein Bajonettverschlusselement in Form einer L-förmigen Nut vor (siehe dazu Fig. 4). Am äußersten proximalen Ende des Kopplungsabschnitts 13 ist eine Schlitzung zu erkennen, die in diesem Abschnitt eine vorbestimmte längsaxiale Elastizität bereitstellt. In Zusammenwirkung mit einer Auflagefläche des Dilatationsinstruments wird diese "strukturintegrierte Feder" vorgespannt und dazu verwendet, den Bajonettverschluss gegen unbeabsichtigtes Öffnen zu sichern. Das Netzmaterial des Schlauchs ist so elastisch, dass er der Ausdehnung des Dilatationswerkzeugs vollständig folgen kann ohne beschädigt zu werden. Bekannte Überzüge, die nur über den Ausdehnungsabschnitt des Dilatationswerkzeugs gezogen werden, etwa Silikon-Überzieher, rutschen nachteilig bei der Ausdehnung sehr leicht wieder herunter und können im schlimmsten Fall im Patienten vergessen werden. Die vorliegende Erfindung ermöglich es, den Überzug 1 dauerhaft sicher mit einem Dilatationsinstrument zu koppeln, da er sich über die vollständige Länge des Schafts erstreckt und erst am Handhabeabschnitt mit dem Dilatationsinstrument verbunden ist, wodurch eine hinreichende Strecke zur längsaxialen Dehnung zum Ausgleich der Querdehnung im Ausdehnungsabschnitt zur Verfügung ist. Dadurch wird bei mechanischen Dilatationsinstrumenten eine gleichmäßig Krafteinleitung in das Gewebe ermöglicht. Mechanische Dilatationsinstrumente haben in der Regel Spreizbacken, die beim Öffnen Spalte freigeben, sodass erstens der Druck auf das Gewebe steigt und zweitens auch die Traumagefahr für das Gewebe durch Einklemmen erhöht ist. Der erfindungsgemäße Überzug 1 verhindert beide Effekte, ohne jedoch die bekannten Nachteile hinsichtlich der Befestigung zu haben. Vorteilhaft kann unter bestimmten Umständen bei Verwendung des erfindungsgemäßen Überzugs 1 sogar auf eine eigene Rückstelleinrichtung des Dilatationsinstruments verzichtet werden, da die Abmessungen und der Werkstoff des Schlauchs so gewählt werden können, dass eine Rückstellkraft erzeugt wird, die auch zum Rückstellen des Dilatationsinstruments ausreicht. Das Netzmaterial des erfindungsgemäßen Überzugs besteht aus einem Gewebe, Geflecht, Gestrick oder Gewirk, bei dem die einzelnen Fäden oder Drähte untereinander nicht verbunden sind, sondern frei gegeneinander verschiebbar sind. Ein zusätzlicher Vorteil des erfindungsgemäßen Überzugs 1 besteht darin, dass die wesentlichen Bauteile des Dilatationsinstruments, nämlich der Schaft und der Ausdehnungsabschnitt, nicht mehr in direktem Kontakt mit Gewebe kommen, sondern nur der Überzug 1. Nach dem Einsatz des Überzugs 1 bei einer Operation wird der Überzug 1 einfach entsorgt, während der Reinigungs- bzw. Desinfektionsaufwand für das Dilatationsinstrument deutlich verringert wird.

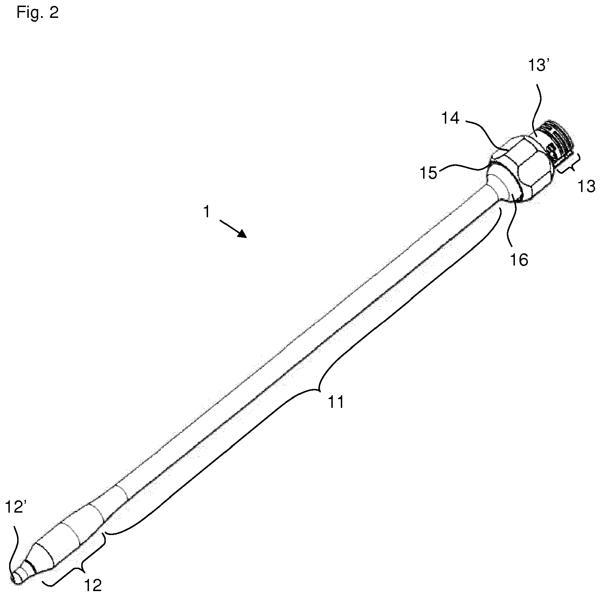

In Fig. 2 ist der Überzug 1 in einem teilaufgeweiteten Zustand dargestellt, wobei das Dilatationsinstrument selbst nicht gezeigt ist. Die Spitze bzw. die distale Endhülse 12' dehnt sich dabei nicht aus, da diese mit dem Schlauch veklebt und / oder vercrimpt ist.

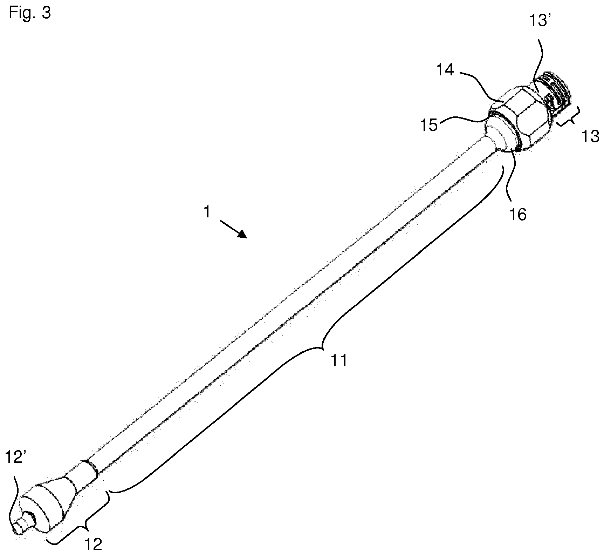

Fig. 3 zeigt den Zustand des Überzugs 1 bei vollständig expandiertem Ausdehnungsabschnitt 12. Das Netzmaterial folgt der Ausdehnung des Dilatationsinstruments elastisch, d. h. kehrt beim Kontrahieren des Dilatationsinstruments stets in den Zustand der Fig. 1 zurück. Das Dilatationsinstrument kann selbstverständlich auch eine andere Ausdehnungs-Geometrie haben, da für unterschiedliche Operations-Typen auch unterschiedliche Ausdehnungs-Geometrien Verwendung finden. Es kann jedoch auch für verschiedene Dilatationswerkzeuge mit unterschiedlichen Ausdehnungs-Geometrien stets der gleiche Überzug 1 verwendet werden.

In Fig. 4 ist das proximale, d. h. das dem Dilatationsinstrument zugewandte, Ende des Überzugs 1 gezeigt, wobei auch noch ein Teil des Schaftabschnitts 11 zu erkennen ist. Im Bereich der Schlauchumlenkung 16 wird der Schlauch von seinem Ruhedurchmesser innerhalb kürzester Strecke radial gedehnt und ist schließlich über der Kopplungshülse 13' gestreift. Der Kopplungsabschnitt 13 hat an seinem proximalen Ende mehrere umlaufende Schlitze 134, die jeweils eine längsaxiale Elastizität in diesem Abschnitt bereitstellen. Insgesamt liegen dort 3 parallele Schlitzreihen vor, wobei zwischen den Schlitzen 133 Stege angeordnet sind, die jedoch umlaufend versetzt sind, sodass sie quasi rechteckfunktionsartig verlaufen, wodurch eine höhere Gesamtelastizität erreicht wird. Die Stege 133 sind jeweils an einer Durchmesserebene, die den Durchmesser und die Längsachse enthält, auf das gegenüberliegende Längsviertel gespiegelt. Das Bajonettverschlusselement wird durch eine L-förmige Einführnut 131 gebildet, die sich parallel der Längsachse erstreckt und die an dem geschlossenen Ende eine Sperrausnehmung 132 hat, in die im Sperrzustand die Sperrnase der Kopplungsvorrichtung des Dilatationswerkzeugs eingreift. Der federnde Abschnitt mit den Schlitzen 134 sorgt hierbei dafür, dass die Sperrnase nicht einfach durch Verdrehen wieder aus der Sperrausnehmung 132 heraus rutschen kann, sondern nur, wenn die Federkraft durch längsaxiales Drücken überwunden wird.

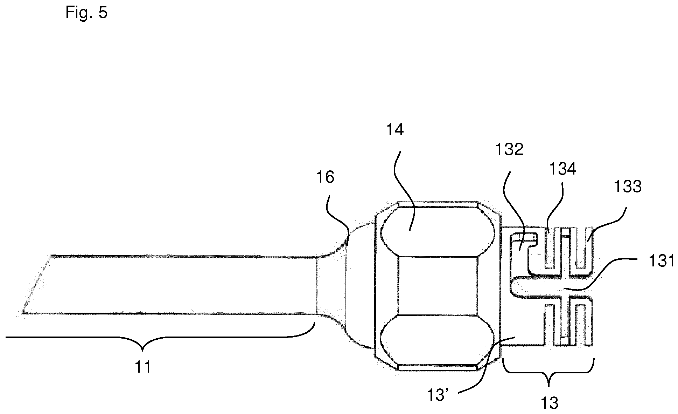

In Fig. 5 ist der Abschnitt des Überzugs 1 aus Fig. 4 in einer Seitenansicht gezeigt, wobei die Ausgestaltung des federnden Abschnitts des Kopplungsabschnitts 13 mit Schlitzen 134 und rechteckfunktionsartig verlaufenden Stegen 133 nochmals gut zu erkennen ist. Die Griffhilfshülse 14 hat Griffflächen 14, die sowohl mit einem Schlüssel gegriffen werden können als auch von Hand ein angenehmes Drehen ermöglichen.

In Fig. 6 ist lediglich die Kopplungshülse 13' perspektivisch dargestellt. Die durch die Schlitzung erreichte federnde Funktion ist im proximalen Endabschnitt 137 umgesetzt, durch den sich auch die Schlitze 134 parallel, teilweise umlaufend und normal zur Längsachse erstrecken. Der distale Endabschnitt 138 ist der Abschnitt der Kopplungshülse 13', über den der schlauchartige Schaftabschnitt des Überzugs 1 (siehe Fig. 1) gestreift wird und über dem auch die Griffhilfshülse 14 (siehe Fig. 5) angeordnet wird. In dem Bereich des distalen Endabschnitts 138 sind drei in gleichem Winkel über den Umfang verteilt angeordnete Rastausnehmungen 136 zu erkennen, mit denen Rastnasen der Griffhilfshülse 14 (siehe Fig. 5) in Eingriff gebracht werden können, so dass die Übertragung des Drehmoments von der Griffhilfshülse auf die Kopplungshülse 13' auch teilweise formschlüssig erfolgen kann, was vorteilhaft ist, da so das Netzmaterial nicht durch Relativbewegungen im Ringspalt zwischen der Kopplungshülse 13' und der Griffhilfshülse 14 (siehe Fig. 5) belastet wird.

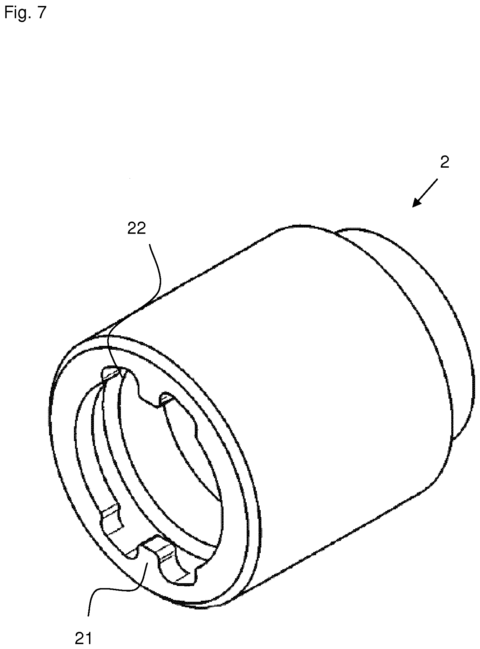

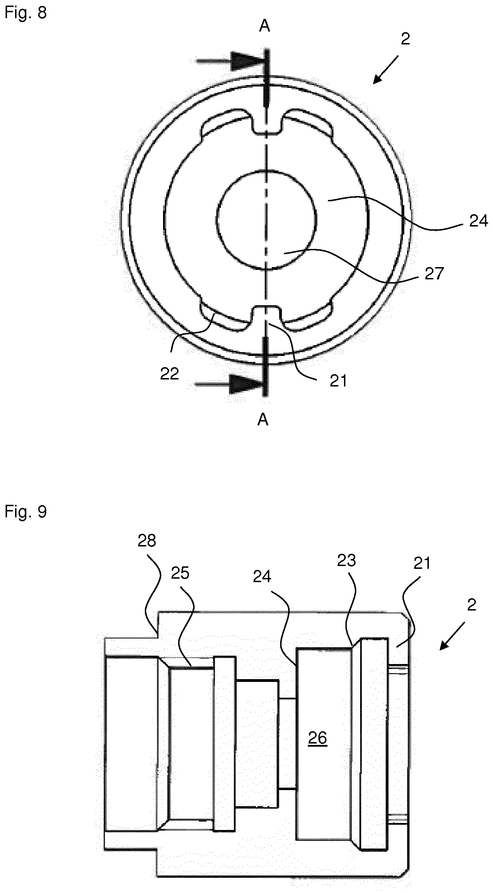

Fig. 7 bis Fig. 9 zeigen die Kopplungsvorrichtung 2, die auch hülsenförmig ist, und in die die Kopplungshülse 13' (siehe Fig. 6) entlang ihrer Längsachse teilweise eingeführt wird. Die Sperrnasen 21 der Kopplungsvorrichtung 2 sind dazu vorgesehen, in die L-förmige Einführnut der Kopplungshülse 13' (siehe Fig. 6) eingeführt zu werden und bis zu deren geschlossenen Ende bewegt zu werden. Die Abmessungen der Sperrnasen 21 korrespondieren mit den Abmessungen der Einführnut 131 und der Sperrausnehmung 132 der Kopplungshülse 13' (siehe Fig. 6). Die Kopplungshülse wird entlang ihres proximalen Endabschnitts, in dem die längsaxiale Elastizität bereitstellt wird, in die Kopplungsvorrichtung 2 eingeführt, wobei sich der proximale Endabschnitt der Kopplungshülse dann im Aufnahmeraum 26 der Kopplungsvorrichtung 2 aufgenommen wird. Die Ausnehmungen 22, die beidseitig der beiden Sperrnasen 21 vorliegen, sind für die Fertigung der Sperrnasen 21. In der Draufsicht der Fig. 8 ist zusätzlich eine Durchtrittsöffnung 27 zu erkennen, durch die der Schaft des Dilatationsinstruments, der die Betätigungsmechanik enthält, geführt wird. Selbstverständlich kann das Dilatationsinstrument auch eine andere Betätigungsart aufweisen, so dass durch die Durchtrittsöffnung 27 etwa auch Hydraulikleitungen, Pneumatikleitungen und/oder elektrische Leitungen geführt sein können. Ferner ist auch eine Schnittebene A-A eingezeichnet, wobei dieser Längsschnitt in Fig. 9 gezeigt ist. Im Längsschnitt ist ein Innengewinde 25 zu erkennen, mit dem die Kopplungsvorrichtung 2 mit dem Dilatationsinstrument verschraubt wird. Ferner ist zu sehen, dass an den Aufnahmeraum 26 für den proximalen Endabschnitt der Kopplungshülse 13' (siehe Fig. 6) sich distal (also hier weiter rechts) ein zylindrischer Abschnitt mit größerem Durchmesser anschließt, wobei dazwischen ein Einführanfang bzw. eine Einführschräge 23 angeordnet ist, die das Einführen der Kopplungshülse in den Aufnahmeraum 26 erleichtern soll. An ihrem proximalen Ende (siehe links in der Figur) hat die Kopplungsvorrichtung 2 einen Abschnitt mit kleinerem Durchmesser und eine Schulter 28, die dazu vorgesehen ist, bündig an einer gegenüberliegenden Fläche eines distalen Endes des Dilatationsinstruments anzuliegen. Es kann vorgesehen sein, dass der proximale Endabschnitt mit dem kleineren Durchmesser in einer zylindrischen Bohrung des Dilatationsinstruments aufgenommen ist und der größere Außendurchmesser dem Außendurchmesser des Dilatationsinstruments entspricht, da so ein harmonisches Gesamtbild des Gesamtinstruments mit gekoppeltem Kopplungsabschnitt erreicht werden kann. An der Schulter 28 kann ein Farbring zur Codierung des Instruments bzw. dessen Stärke, Charge oder einer anderen Eigenschaft angebracht sein.

Ein solches Dilatationsinstrument 3 mit angekoppelter Kopplungsvorrichtung 2 ist in Fig. 10 dargestellt. Das Instrument 3 kann in drei Abschnitte 31, 32, 35 unterteilt werden, einen Handhabeabschnitt 35, einen Schaftabschnitt 31 und einen Ausdehnungsabschnitt 32. Am proximalen Ende des Handhabeabschnitts 35 ist ein Kraftübertragungselement mit endseitigem Betätigungsknauf 34 angeordnet, die in Wirkverbindung mit dem Ausdehnungsabschnitt 32 steht, etwa durch einen Seilzug oder eine Schub- / Zugstange, die im Inneren des Schafts 31 geführt ist. Durch Herausziehen des Knaufs 34 werden die Spreizbacken 321 mittels der Schubstange betätigt, woraufhin sich die Spreizbacken 321 in radialer Richtung ausdehnen. Die Kopplungsvorrichtung 2 ist mit dem distalen Ende des Handhabeabschnitts 35 verschraubt bzw. verklebt, wobei die Außendurchmesser der Kopplungsvorrichtung 2 und des Handhabeabschnitts 35 in etwa gleich sind, so dass sich ein harmonischer Übergang ergibt, der ergonomisch angenehm ist. Vor der Durchführung einer Operation mit dem Dilatationsinstrument 3 wird ein erfindungsgemäßer Überzug (siehe Fig. 1) über den Ausdehnungsabschnitt 32 und den Schaft 31 gezogen. Die Kopplungshülse 13' mit dem federnden proximalen Endabschnitt des Überzugs 1 (siehe Fig. 1) wird mit der Einführnut in die Sperrnasen der Kopplungsvorrichtung 2 eingefädelt und nach dem Erzeugen einer Vorspannung des federnden Abschnitts bis zur Sperrausnehmung der Kopplungshülse verdreht. Nach dem Loslassen der Kopplungshülse ist der Überzug durch die Zusammenwirkung der Vorspannung des federnden Abschnitts der Kopplungshülse mit der Sperrausnehmung und der Sperrnase gesichert und kann nicht mehr ungewollt vom Dilatationsinstrument rutschen. Der Überzug deckt in gekoppeltem Zustand den Schaft 31 und den Ausdehnungsabschnitt 32 des Dilatationsinstruments vollständig ab. Vorteilhaft ist das Netzmaterial des Überzugs so elastisch, dass es der Ausdehnung des Ausdehnungsabschnitts des Dilatationsinstruments 3 folgen kann und sogar noch eine Rückstellkraft auf die Spreizbacken 321 ausübt. Ein Dilatationsinstrument 3 mit darüber gestreiften Überzug kann im Klinikalltag nach der Operation mit viel weniger Aufwand gereinigt und desinfiziert werden, das das eigentliche Instrument gar nicht in Kontakt mit Patientengewebe kommt, sondern nur der Überzug.

Die Funktion des Betätigungsmechanismus des Dilatationsinstruments 3 wird anhand der Fig. 11 knapp beschrieben. Im Inneren des Schaftabschnitts 31 verläuft eine Zugstange 312, die in einem Hüllrohr 312 geführt ist. Die Zugstange 312 erstreckt sich vom Ausdehnungsabschnitt 32 bis zum äußersten distalen Ende des Handhabeabschnitts 35, an dem sie einen Knauf 34 aufweist. An dem Knauf 34 kann die Zugstange 312 direkt händisch betätigt werden oder es kann eine Betätigungshilfsvorrichtung, etwa mit einem Pinzetten- oder Pistolengriff, damit in Eingriff gebracht werden. Im Ausdehnungsabschnitt 32 sind Spreizbacken 321 zu erkennen, die an dem Drehpunkt 323 angelenkt sind. In dieser Längsschnittsansicht sind im Wesentlichen nur zwei Spreizbacken 321 zu erkennen, die sich in der Bildebene bewegen lassen, jedoch weist das Instrument weitere umfänglich verteilte Spreizbacken auf, die eine dreidimensionale Aufweitung des Ausdehnungsabschnitts 32 ermöglichen. Am distalen Ende der Zugstande 312 ist im Ausdehnungsabschnitt 32 eine Kugel 322 angebracht, die bei der Betätigung relativ zu den Spreizbacken 321 verschoben wird und auf einer schiefen Führungsbahn der Spreizbacken 321 abgleitet, wodurch die Spreizbacken auseinander gedrückt werden. Im Handhabeabschnitt 35 ist ferner ein Zuführport 33 angeordnet, durch den flüssige oder gasförmige Medien zum Spülen oder Reinigen des Dilatationsinstruments 3 zugeführt werden können. Damit während des Betriebs keine Fremdkörper in den Zuführport 33 eindringen ist dieser mit einer Kappe 33' verschlossen, die über eine Lasche verliersicher befestigt ist.

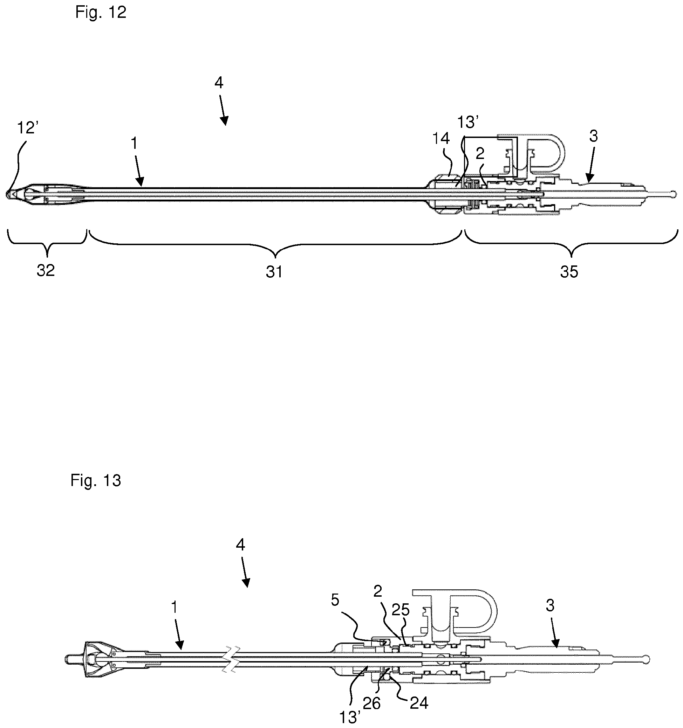

In Fig. 12 ist das in Fig. 11 gezeigt Dilatationsinstrument 3 mit gekoppeltem Überzug in einem Längsschnitt dargestellt; es handelt sich ergo um die erfindungsgemäße Dilatationsvorrichtung 4. Der Überzug 1 ist mit der Kopplungshülse 13' lösbar mit der Kopplungsvorrichtung 2 gekoppelt, die wiederum lösbar mit dem distalen Ende des Handhabeabschnitts 35 des Dilatationsinstruments 3 verbunden ist. Der Überzug 1 erstreckt sich schlauchartig und eng anliegend entlang des ganzen Schaftabschnitts 31 des Dilatationsinstruments 3 und auch über den Ausdehnungsabschnitt 32. Am äußersten distalen Ende der Dilatationsvorrichtung 4 ist der Überzug mit der Endhülse 12' geschlossen. Einer Ausdehnung des Ausdehnungsabschnitts 32 kann der Überzug 1 aufgrund seiner Netzstruktur und guten Elastizität stets eng anliegend folgen. Der Zustand der Dilatationsvorrichtung mit vollständig expandiertem Ausdehnungsabschnitt 32 ist in Fig. 13 dargestellt.

Als Alternative zu dem federnden proximalen Endabschnitt der Kopplungshülse 13' kann die Kopplungshülse 13' auch über ihre komplette Länge starr sein, wobei die gewünschte federnde Funktion durch ein Zusatzbauteil erreicht wird. Eine solche Ausführungsform ist in Fig. 13 dargestellt. Hier ist zwischen der Auflagestirnfläche 24, der Kopplungsvorrichtung 2 und der gegenüberliegenden distalen Anlagefläche der Kopplungshülse 13' ein O-Ring 5 angeordnet, der die Elastizität bereitstellt, die die Sperrnase der Kopplungsvorrichtung in die Sperrausnehmung der Kopplungshülse drückt. Es können aber auch andere federnde Bauteile zum Einsatz kommen, beispielsweise eine Feder, insbesondere eine Schraubenfeder, oder eine Gasfüllung, was jedoch nicht figurativ gezeigt ist. Auch in dieser Ausführungsform ist die Kopplungsvorrichtung 2 lösbar mittels eines Gewindes 25 mit dem distalen Ende des Handhabeabschnitts 35 (siehe Fig. 12) der Dilatationsvorrichtung 3 verbunden und die Kopplungshülse 13' teilweise vom Aufnahmeraum 26 aufgenommen. Die Ausführungsform mit O-Ring 5 anstatt federnder Kopplungshülse 13' bietet den Vorteil, dass der Überzug 1 günstiger bereitgestellt werden kann; es muss keine komplexe Schlitzstruktur gefertigt werden, sondern es wird nur ein standardisierter O-Ring 5 eingesetzt.

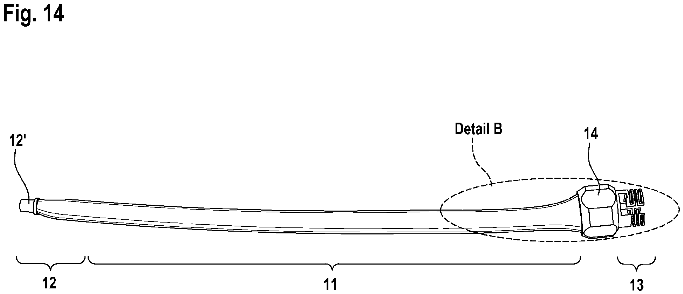

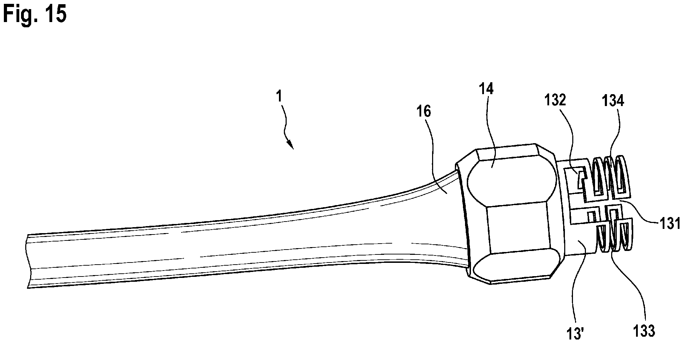

In den Fig. 14 und Fig. 15 ist eine weitere Draufsicht auf den erfindungsgemäßen Überzug 1 dargestellt, wobei f das Detail B aus Fig. 14 zeigt. Es ist hierbei gut die Netzstruktur in dem Schaftabschnitt 11 zu erkennen Auch sind die Schlüsselflächen der Griffhilfshülse 14 sowie die Einführnut 131, die Sperrausnehmung 132 und die Elemente des federnden Abschnitts, Schlitze 134 und Stege 133, dargestellt.

### BEZUGSZEICHENLISTE

- Nr.: Beschreibung
- 1: Überzug für Dilatationsinstrument
- 11: Schaftabschnitt des Überzugs
- 12: Distaler Ausdehnungsabschnitt des Überzugs
- 12': Endhülse des Überzugs
- 13: Proximaler Kopplungsabschnitt des Überzugs
- 13': Kopplungshülse
- 131: Einführausnehmung der Kopplungshülse
- 132: Sperrausnehmung der Kopplungshülse
- 133: Steg der Kopplungshülse
- 134: Schlitz der Kopplungshülse
- 136: Rastausnehmung der Kopplungshülse
- 137: Proximaler Endabschnitt der Kopplungshülse
- 138: Distaler Endabschnitt der Kopplungshülse
- 14: Griffhilfshülse
- 15: Ringspalt zwischen Griffhilfshülse und Kopplungshülse
- 16: Schlauchumlenkung
- 2: Kopplungsvorrichtung des Dilatationsinstruments
- 21: Sperrnase der Kopplungsvorrichtung
- 22: Ausnehmung für Montagewerkzeug der Kopplungsvorrichtung
- 23: Einführanfasung der Kopplungsvorrichtung
- 24: Proximale Auflagestirnfläche der Kopplungsvorrichtung
- 25: Innengewinde der Kopplungsvorrichtung
- 26: Aufnahmeraum der Kopplungsvorrichtung
- 27: Durchtrittsöffnung der Kopplungsvorrichtung
- 28: Proximale Schulter der Kopplungshülse
- 3: Dilatationsinstrument
- 31: Schaft des Dilatationsinstruments
- Nr.: Beschreibung
- 32: Ausdehnungsabschnitt des Dilatationsinstruments
- 321: Spreizbacken des Dilatationsinstruments
- 33: Zuführport des Dilatationsinstruments
- 33': Deckel mit Schlaufe des Zuführports
- 34: Kraftübertragungselement
- 35: Handhabeabschnitt des Dilatationsinstruments
- 4: Dilatationsvorrichtung
- 5: O-Ring

## Patentansprüche

1. Dilatationsvorrichtung (4) umfassend
- ein Dilatationsinstrument (3), das einen distalen radial ausdehnbaren Abschnitt (32), der über einen länglichen Schaft (31) mit einem Handhabeabschnitt (35) gekoppelt ist, aufweist und
- einen aufweitbaren Überzug (1) für den Ausdehnungsabschnitt (32) des Dilatationsinstruments (3), der ein geschlossenes distales Ende und ein offenes proximales Ende hat, wobei
der Überzug (1) einen Schaftabschnitt (11) aufweist, der sich entlang des Schafts (31) des Dilatationsinstruments (3) erstreckt, wobei das offene proximale Ende des Überzugs (1) lösbar mit einem distalen Ende des Handhabeabschnitts (32) des Dilatationsinstruments (3) gekoppelt ist,
**dadurch gekennzeichnet, dass**
der Überzug (1) aus einem Netzmaterial besteht, das aus einem Gewebe, Geflecht, Gestrick oder Gewirk besteht, bei dem die einzelnen Fäden oder Drähte untereinander nicht verbunden sind, sondern frei gegeneinander verschiebbar sind.

2. Dilatationsvorrichtung (4) nach Anspruch 1,
**dadurch gekennzeichnet, dass**
das Netzmaterial ein Drahtnetzmaterial, bevorzugt ein Drahtnetzmaterial aus einem biokompatiblen Edelstahl ist.

3. Dilatationsvorrichtung (4) nach Anspruch 1 oder 2,
**dadurch gekennzeichnet, dass**
- der Schaftabschnitt (11) des Überzugs (1) an dem offenen proximalen Ende mit einer Kopplungshülse (13') verbunden ist, bevorzugt verklebt, und
- mit dem distalen Ende des Handhabeabschnitts (35) des Dilatationsinstruments eine Kopplungsvorrichtung (2) verbunden ist, bevorzugt lösbar verbunden, besonders bevorzugt über ein Gewinde verbunden, wobei die Kopplungshülse (13') des Überzugs (1) lösbar mit der Kopplungsvorrichtung (2) des Dilatationsinstruments (3) gekoppelt ist.

4. Dilatationsvorrichtung (4) nach Anspruch 3,
**dadurch gekennzeichnet, dass**
- die Kopplungshülse (13') ein Bajonettverschlusselement aufweist, das bevorzugt eine im Wesentlichen L-förmige Einführausnehmung (131) aufweist, die sich von einer proximalen Stirnfläche längsaxial in einer Wandung erstreckt, wobei an einem geschlossenen Ende der Einführausnehmung (131) eine Sperrausnehmung (132) vorliegt und
- die Kopplungsvorrichtung (2) zumindest eine sich radial nach innen erstreckende Sperrnase (21) aufweist, deren Abmessungen mit der Einführausnehmung (131) der Kopplungshülse (13') korrespondieren,
wobei die Sperrnase (21) in einem Sperrzustand von der Sperrausnehmung (132) der Kopplungshülse (13') aufgenommen ist.

5. Dilatationsvorrichtung (4) nach Anspruch 3 oder 4,
**dadurch gekennzeichnet, dass**
zwischen einer proximalen Stirnfläche der Kopplungshülse (13') und einer distalen Stirnfläche der Kopplungsvorrichtung (23) ein Federelement angeordnet ist, bevorzugt eine Druckfeder, besonders bevorzugt eine federnde Polymerscheibe, am meisten bevorzugt ein O-Ring (5).

6. Dilatationsvorrichtung (4) nach zumindest einem der Ansprüche 3 bis 5,
**dadurch gekennzeichnet, dass**
- die Kopplungsvorrichtung (2) einen Aufnahmeraum (26) für die Kopplungshülse (13') des Überzugs (1) aufweist, der sich längsaxial von einer distalen Stirnfläche der Kopplungsvorrichtung (2) in Richtung des proximalen Endes erstreckt, wobei die Kopplungshülse (13') zumindest entlang eines proximalen Endabschnitts (137) darin aufgenommen ist, und wobei an den Aufnahmeraum (26) proximal eine Auflagestirnfläche (24) angrenzt, an der die Kopplungshülse (13') abgestützt ist.

7. Dilatationsvorrichtung (4) nach zumindest einem der Ansprüche 3 bis 6,
**dadurch gekennzeichnet, dass**
- an der Kopplungsvorrichtung (2) umfänglich benachbart, bevorzugt beidseitig, zu der zumindest einen Sperrnase (21) zumindest eine sich radial nach außen erstreckende Montageausnehmung (22) vorliegt, und/oder
- die Kopplungsvorrichtung (2) an einem distalen Ende des Aufnahmeraums (26) eine Einführanfasung (23) aufweist.

8. Dilatationsvorrichtung (4) nach zumindest einem der Ansprüche 1 bis 7,
**dadurch gekennzeichnet, dass**
der Überzug (1) aus einem Netzmaterial besteht und einen Schaftabschnitt (11) aufweist, der an dem offenen proximalen Ende mit einer Kopplungshülse (13') verbunden ist.

9. Dilatationsvorrichtung (4) nach Anspruch 8,
**dadurch gekennzeichnet, dass**
die Kopplungshülse (13') in einem proximalen Endabschnitt (137) längsaxial elastisch ist, wobei die Kopplungshülse (13') in dem proximalen Endabschnitt (137) bevorzugt zumindest einen zumindest teilweise umlaufenden Schlitz (134) aufweist.

10. Dilatationsvorrichtung (4) nach Anspruch 9,
**dadurch gekennzeichnet, dass**
die Kopplungshülse (13') in dem proximalen Endabschnitt (137) eine Mehrzahl von parallelen Schlitzen (134), die normal zu der Längsachse ausgerichtet sind, aufweist, wobei zwischen den Schlitzen (134) Stege (133) vorliegen, die in der Abwicklung bevorzugt alternierend, wellenförmig oder rechteckfunktionsartig verlaufen, wobei die Kopplungshülse (13') bevorzugt zumindest zwei Ringsektoren aufweist, und wobei in benachbarten Ringsektoren die Stege (133) jeweils gespiegelt angeordnet sind.

11. Dilatationsvorrichtung (4) nach zumindest einem der Ansprüche 8 bis 10,
**dadurch gekennzeichnet, dass**
über einem distalen Endabschnitt (138) der Kopplungshülse (13') eine Griffhilfshülse (14) angeordnet ist, bevorzugt eine Griffhilfshülse (14) mit einem Schlüsselansatz oder einer Rändelung, die drehfest mit dem distalen Endabschnitt (138) verbunden ist, bevorzugt verklebt.

12. Dilatationsvorrichtung (4) nach Anspruch 11,
**dadurch gekennzeichnet, dass**
die Kopplungshülse (13') in einer Wand zumindest eine Rastausnehmung (136) aufweist, und die Griffhilfshülse (14) korrespondierende Rastnasen aufweist, wobei die Rastnasen der Griffhilfshülse (14) in Eingriff mit den Rastausnehmungen (136) der Kopplungshülse (13') stehen.

13. Dilatationsvorrichtung (4) nach zumindest einem der Ansprüche 8 bis 12,
**dadurch gekennzeichnet, dass**
über dem distalen geschlossenen Ende des Überzugs (1) eine Endhülse (12') angeordnet ist, die bevorzugt mit dem distalen Ende verklebt und/oder verquetscht ist.

14. Dilatationsvorrichtung (4) nach zumindest einem der Ansprüche 8 bis 13,
**dadurch gekennzeichnet, dass**
- das Netzmaterial ein Gewirke, Gestrick oder Geflecht ist und / oder
- der Werkstoff des Netzmaterials einen Durchmesser von 0,08 mm aufweist und / oder
- der Werkstoff des Netzmaterials ein Metall oder eine Metalllegierung, bevorzugt eine biokompatibler Edelstahl oder eine Formgedächtnislegierung, ist.

## Claims

1. A dilation device (4) comprising
- a dilation instrument (3) which has a distal radially extendible section (32) which is coupled to a handle section (35) via an elongated shaft (31) and
- an expandable cover (1) for the extension section (32) of the dilation instrument (3), which has a closed distal end and an open proximal end, wherein
the cover (1) has a shaft section (11) which extends along the shaft (31) of the dilation instrument (3), wherein the open proximal end of the cover (1) is releasably coupled to a distal end of the handle section (32) of the dilation instrument (3),
**characterised in that**
the cover (1) consists of a mesh material which consists of a woven, braided, pleated or knitted fabric in which the individual threads or wires are not connected to one another but can be freely moved relative to one another.

2. The dilation device (4) according to claim 1,
**characterised in that**
the mesh material is a wire mesh material, preferably a wire mesh material of a biocompatible stainless steel.

3. The dilation device (4) according to claim 1 or 2,
**characterised in that**
- the shaft section (11) of the cover (1) is connected, preferably glued, at the open proximal end to a coupling sleeve (13') and
- a coupling device (2) is connected, preferably releasably connected, particularly preferably connected via a thread, to the distal end of the handle section (35) of the dilation instrument,
wherein the coupling sleeve (13') of the cover (1) is releasably coupled to the coupling device (2) of the dilation instrument (3).

4. The dilation device (4) according to claim 3,
**characterised in that**
- the coupling sleeve (13') has a bayonet closure element, which preferably has a substantially L-shaped insertion recess (131) which extends longitudinally axially in a wall from a proximal end face, wherein a locking recess (132) is present at a closed end of the insertion recess (131) and
- the coupling device (2) has at least one locking lug (21) extending radially inwards, the dimensions of which correspond to the insertion recess (131) of the coupling sleeve (13'),
wherein the locking lug (21) is received in a locked state by the locking recess (132) of the coupling sleeve (13').

5. The dilation device (4) according to claim 3 or 4,
**characterised in that**
a spring element, preferably a compression spring, particularly preferably a resilient polymer disc, most preferably an O-ring (5), is arranged between a proximal end face of the coupling sleeve (13') and a distal end face of the coupling device (23).

6. The dilation device (4) according to at least one of claims 3 to 5,
**characterised in that**
- the coupling device (2) has a receiving space (26) for the coupling sleeve (13') of the cover (1), which extends longitudinally axially from a distal end face of the coupling device (2) in the direction of the proximal end, wherein the coupling sleeve (13') is received therein at least along a proximal end section (137), and wherein a support end face (24), on which the coupling sleeve (13') is supported, adjoins the receiving space (26) proximally.

7. The dilation device (4) according to at least one of claims 3 to 6,
**characterised in that**
- at least one mounting recess (22) extending radially outwards is present on the coupling device (2) circumferentially adjacent to, preferably on both sides of, the at least one locking lug (21), and/or
- the coupling device (2) has an insertion chamfer (23) at a distal end of the receiving space (26).

8. The dilation device (4) according to at least one of claims 1 to 7,
**characterised in that**
the cover (1) consists of a mesh material and has a shaft section (11) which is connected to a coupling sleeve (13') at the open proximal end.

9. The dilation device (4) according to claim 8,
**characterised in that**
the coupling sleeve (13') is longitudinally axially elastic in a proximal end section (137), wherein the coupling sleeve (13') preferably has at least one at least partially circumferential slot (134) in the proximal end section (137).

10. The dilation device (4) according to claim 9,
**characterised in that**
the coupling sleeve (13') in the proximal end section (137) has a plurality of parallel slots (134) which are aligned normal to the longitudinal axis, wherein webs (133) are present between the slots (134), which, in a developed view, preferably run alternating, wave-shaped or like a rectangular function, wherein the coupling sleeve (13') preferably has at least two ring sectors, and wherein the webs (133) are each arranged in a mirrored manner in adjacent ring sectors.

11. The dilation device (4) according to at least one of claims 8 to 10,
**characterised in that**
an auxiliary grip sleeve (14) is arranged over a distal end section (138) of the coupling sleeve (13'), preferably an auxiliary grip sleeve (14) with a key attachment or a knurl, which is non-rotatably connected, preferably glued, to the distal end section (138).

12. The dilation device (4) according to claim 11,
**characterised in that**
the coupling sleeve (13') has at least one latching recess (136) in a wall, and the auxiliary grip sleeve (14) has corresponding latching lugs, wherein the latching lugs of the auxiliary grip sleeve (14) are in engagement with the latching recesses (136) of the coupling sleeve (13').

13. The dilation device (4) according to at least one of claims 8 to 12,
**characterised in that**
an end sleeve (12'), which is preferably glued to and/or crimped with the distal end, is arranged over the distal closed end of the cover (1).

14. The dilation device (4) according to at least one of claims 8 to 13,
**characterised in that**
- the mesh material is a knitted, pleated or braided fabric and/or
- the material of the mesh material has a diameter of 0.08 mm and/or
- the material of the mesh material is a metal or a metal alloy, preferably a biocompatible stainless steel or a shape memory alloy.

## Revendications

1. Dispositif de dilatation (4) comprenant
- un instrument de dilatation (3) qui possède une section distale (32) extensible radialement qui est accouplée à une section de poignée (35) par l'intermédiaire d'une tige allongée (31) et
- un couvercle (1) extensible pour la section d'expansion (32) de l'instrument de dilatation (3), qui a une extrémité distale fermée et une extrémité proximale ouverte, dans lequel
le couvercle (1) présente une section de tige (11) qui s'étend le long de la tige (31) de l'instrument de dilatation (3), dans lequel l'extrémité proximale ouverte du couvercle (1) est accouplée de manière amovible à une extrémité distale de la section de poignée (32) de l'instrument de dilatation (3),
**caractérisé en ce que**
la couverture (1) est constituée d'un matériau en filet qui est constitué d'un tissu, tresse, tricot ou maille, dans lequel les fils ou fils métalliques individuels ne sont pas reliés les uns aux autres mais peuvent être librement déplacés les uns par rapport aux autres.

2. Dispositif de dilatation (4) selon la revendication 1,
**caractérisé en ce que**
le matériau en filet est un matériau en filet en fils métalliques, de préférence un matériau en filet de fils métalliques constitué d'un acier inoxydable biocompatible.

3. Dispositif de dilatation (4) selon la revendication 1 ou 2,
**caractérisé en ce que**
- la section de tige (11) du couvercle (1) est reliée, de préférence collée, au niveau de l'extrémité proximale ouverte à un manchon d'accouplement (13') et
- un dispositif d'accouplement (2) est relié à l'extrémité distale de la section de poignée (35) de l'instrument de dilatation, de préférence relié de manière amovible, de manière particulièrement préférée par l'intermédiaire d'un filetage,
dans lequel le manchon d'accouplement (13') du couvercle (1) est accouplé de manière amovible au dispositif d'accouplement (2) de l'instrument de dilatation (3).

4. Dispositif de dilatation (4) selon la revendication 3,
**caractérisé en ce que**
- le manchon d'accouplement (13') présente un élément de verrouillage à baïonnette, qui présente de préférence un évidement d'insertion (131) sensiblement en forme de L qui s'étend longitudinalement axialement dans une paroi à partir d'une face frontale proximale, dans lequel au niveau d'une extrémité fermée de l'évidement d'insertion (131) se trouve un évidement de verrouillage (132) et
- le dispositif d'accouplement (2) présente au moins une patte de verrouillage (21) s'étendant radialement vers l'intérieur, dont les dimensions correspondent à l'évidement d'insertion (131) du manchon d'accouplement (13'),
dans lequel la patte de verrouillage (21) est reçue dans un état verrouillé par l'évidement de verrouillage (132) du manchon d'accouplement (13').

5. Dispositif de dilatation (4) selon la revendication 3 ou 4,
**caractérisé en ce que**
un élément à ressort est agencé entre une face frontale proximale du manchon d'accouplement (13') et une face frontale distale du dispositif d'accouplement (23), de préférence un ressort de compression, de manière particulièrement préférée un disque polymère élastique, le plus préférablement un joint torique (5).

6. Dispositif de dilatation (4) selon au moins l'une des revendications 3 à 5,
**caractérisé en ce que**
- le dispositif d'accouplement (2) présente un espace de réception (26) pour le manchon d'accouplement (13') du couvercle (1), qui s'étend longitudinalement axialement depuis une face frontale distale du dispositif d'accouplement (2) en direction de l'extrémité proximale, dans lequel le manchon d'accouplement (13') est logé à l'intérieur au moins le long d'une section d'extrémité proximale (137), et dans lequel une face frontale de support (24) jouxte l'espace de réception (26) de manière proximale, sur laquelle face le manchon d'accouplement (13') est supporté.

7. Dispositif de dilatation (4) selon au moins l'une des revendications 3 à 6,
**caractérisé en ce que**
- adjacent circonférentiellement au dispositif d'accouplement (2), de préférence des deux côtés, auquel au moins une patte de verrouillage (21) présente au moins un évidement de montage (22) s'étendant radialement vers l'extérieur, et/ou
- le dispositif d'accouplement (2) présente un chanfrein d'insertion (23) au niveau d'une extrémité distale de l'espace de réception (26).

8. Dispositif de dilatation (4) selon au moins l'une des revendications 1 à 7,
**caractérisé en ce que**
le couvercle (1) est constitué d'un matériau en filet et présente une section de tige (11) qui est reliée à un manchon d'accouplement (13') au niveau de l'extrémité proximale ouverte.

9. Dispositif de dilatation (4) selon la revendication 8,
**caractérisé en ce que**
le manchon d'accouplement (13') est élastique longitudinalement et axialement dans une section d'extrémité proximale (137), le manchon d'accouplement (13') présentant de préférence au moins une fente au moins partiellement circonférentielle (134) dans la section d'extrémité proximale (137).

10. Dispositif de dilatation (4) selon la revendication 9,
**caractérisé en ce que**
le manchon d'accouplement (13') dans la section d'extrémité proximale (137) présente une pluralité de fentes parallèles (134) qui sont alignées perpendiculairement à l'axe longitudinal, dans lequel des entretoises (133) sont présentes entre les fentes (134), qui, dans le tracé, sont de préférence alternées, ondulées ou à fonction rectangulaire, dans lequel le manchon d'accouplement (13') présente de préférence au moins deux secteurs d'anneau et dans lequel les entretoises (133) sont agencées en miroir respectivement dans des secteurs d'anneau adjacents.

11. Dispositif de dilatation (4) selon au moins l'une des revendications 8 à 10,
**caractérisé en ce que**
un manchon de préhension auxiliaire (14) est agencé sur une section d'extrémité distale (138) du manchon d'accouplement (13'), de préférence un manchon de préhension auxiliaire (14) avec une fixation de clé ou une molette, qui est reliée de manière non rotative à la section d'extrémité distale (138), de préférence collée.

12. Dispositif de dilatation (4) selon la revendication 11,
**caractérisé en ce que**
le manchon d'accouplement (13') présente au moins un évidement de verrouillage (136) dans une paroi, et le manchon de préhension auxiliaire (14) présente des pattes de verrouillage correspondantes, dans lequel les pattes de verrouillage du manchon de préhension auxiliaire (14) sont en prise avec l'évidement de verrouillage (136) du manchon d'accouplement (13').

13. Dispositif de dilatation (4) selon au moins l'une des revendications 8 à 12,
**caractérisé en ce que**
un manchon d'extrémité (12') est agencé sur l'extrémité distale fermée du couvercle (1), qui est de préférence collée et/ou sertie à l'extrémité distale.

14. Dispositif de dilatation (4) selon au moins l'une des revendications 8 à 13,
**caractérisé en ce que**
- le matériau en filet est une maille, un tricot ou une tresse et/ou
- le matériau du matériau en filet présente un diamètre de 0,08 mm et/ou
- le matériau du matériau en filet est un métal ou un alliage métallique, de préférence un acier inoxydable biocompatible ou un alliage à mémoire de forme.
